# EUROPÄISCHE PATENTSCHRIFT

(11) **EP 1 574 170 B1**
(45) Veröffentlichungstag und Bekanntmachung des Hinweises auf die Patenterteilung: **11.04.2007**
(21) Anmeldenummer: 04005440.5
(22) Anmeldetag: 08.03.2004
(51) Int. Cl.: A61B 17/15

(54) **Justierbares Bearbeitungshilfsmittel für die Knochenbearbeitung**
Adjustable working aid for treatment of bones
Outil ajustable pour l'aide au travail des os

(43) Veröffentlichungstag der Anmeldung: 14.09.2005
(73) Patentinhaber: BrainLAB AG, 85551 Kirchheim/Heimstetten (DE)
(72) Erfinder: Neubauer, Timo, 85622 Feldkirchen (DE); Plassky, Norman, 99099 Erfurt (DE); Saffari, Anusch, 83666 Schaftlach (DE)
(74) Vertreter: Schwabe - Sandmair - Marx

(56) Entgegenhaltungen:
- EP-A- 1 249 213
- WO-A-20/04017843
- DE-A- 10 207 035
- DE-U- 20 219 357
- US-A- 5 833 693
- US-A- 6 030 211

## Beschreibung

Die vorliegende Erfindung betrifft ein justierbares Bearbeitungshilfsmittel für die Führung und/oder Positionierung eines Werkzeugs zur Bearbeitung eines Knochens, insbesondere zur Führung eines Schneidwerkzeuges oder Bohrers zur Knochen-Resektion und/oder zum Führen und/oder Positionieren eines medizinischen Operationsinstruments.

Beim Anbringen von Implantaten, wie zum Beispiel künstlichem Knie-, Ellbogen-, Finger- oder Hüftgelenken ist es erforderlich, das Implantat, wie zum Beispiel ein Gelenk oder ein Knochenteil, möglichst genau am angrenzenden Knochen zu positionieren. Hierzu müssen möglichst exakte Schnitte oder auch Bohrungen vorgenommen werden, und zwar insbesondere an den an das Gelenk angrenzenden Knochenstrukturen.

Insbesondere bei der Knie-Arthroplastik ist ein präziser Schnitt erforderlich, um eine maximale Stabilität und Beständigkeit des Implantats zu erzielen. Dazu muss der Schneidblock, der als Schablone für den Schneidvorgang dient, möglichst genau platziert werden, um eine exakte Resektion zu erzielen. Bei Verwendung von CAS (Computer Assisted Surgery) ist die Knochenstruktur normalerweise einem Navigationssystem nach einem Einleseverfahren bekannt. Dies ermöglicht es dem Chirurgen, Stäbe zu vermeiden, die extern oder intramedullär, also im Knochenmark, zur Stabilisierung verlaufen, um die Schneidblöcke relativ zur Knochenstruktur exakt zu platzieren. Wird hierauf verzichtet, so muss aber dennoch der Schneidblock exakt platziert werden. Durch die Verwendung von CAS kann der Schneidblock freihändig angebracht werden. Hier hängt dann aber die Genauigkeit der Platzierung von dem Können des Chirurgen ab.

Aus WO 2004/017843 A1 ist ein Führungsblock zur Verwendung bei chirurgischen Eingriffen bekannt. Dieser Block dient dazu chirurgische Werkzeuge genau relativ zu einem anatomischen Merkmal zu lokalisieren. Ein Fixierungsteil kann direkt am Gewebe des Patienten befestigt werden.

Aus US 6,030,211 ist ein Führungsapparat für Instrumente bekannt. Der Anwendungsbereich befindet sich insbesondere in der Zahnmedizin. Der Apparat umfasst einen Instrumentenhalter, der in drei räumliche Richtungen verlagerbar ist.

Aus US 5,833,693 ist eine Führung für einen Bohrer bekannt. Mittels eines Stiftes kann die Führung lokalisiert werden. Ein Führungsblock ist mechanisch mit dem Lokalisierungsstift unten und einem Durchgangsloch versehen, um dadurch einen Bohrer zu führen. Der Führungsblock ist relativ zum Lokalisierungsstift beweglich.

Aus DE 202 19 357 U1 ist eine Führungseinrichtung für ein chirurgisches Bearbeitungswerkzeug bekannt. Ein Führungselement führt das Bearbeitungswerk. Das Führungselement ist an der Führungseinrichtung nur in einer Ebene verschiebbar gelagert. Das Führungselement ist über mindestens einen Lenker mittels eines Drehgelenks mit einem Rahmen der Führungseinrichtung verbunden, dessen Drehachse senkrecht auf der Führungsebene steht.

Aus DE 10 207 035 A1 ist eine Schablone zur Führung eines chirurgischen Bearbeitungswerkzeuges bekannt. Die Schablone dient zur Führung eines chirurgischen Bearbeitungswerkzeuges mit einer Befestigungseinrichtung zur Festlegung der Schablone auf einem zu bearbeitenden Knochen. Die Befestigungseinrichtung umfasst drei Knochenschrauben mit jeweils einem in einem Knochen einschraubbaren Schraubabschnitt und einem Abstand dazu angeordneten Halteabschnitt. Der Schraubabschnitt und Halteabschnitt können relativ zueinander festgeklemmt werden.

Aus EP 1 249 213 A2 ist ein Verfahren und eine Vorrichtung zur präoperativen Bestimmung der Positionsdaten von Endoprothesenteilen bekannt. Dabei wird eine Sägeschablone verwendet, die ein Markierungselement trägt. Die Lage einer Führungsfläche für ein Sägeblatt relativ zum Markierungselement wird mit einem handgeführten Tastelement abgebildet, indem das Tastelement mit seiner Spitze an der Führungsfläche entlang geführt wird. Weitere Markierungselemente sind in Knochen eingeschraubt.

Aufgabe der Erfindung ist es, ein Bearbeitungshilfsmittel bereitzustellen, das eine exakte Bearbeitung eines Knochens ermöglicht.

Vorgenannte Aufgabe wird durch das Bearbeitungshilfsmittel nach Anspruch 1 gelöst. Vorteilhafte Weiterbildungen gehen aus den Unteransprüchen hervor.

Vorteilhaft wird ein justierbares Bearbeitungshilfsmittel für die Bearbeitung eines Knochens bereitgestellt. Dieses Bearbeitungshilfsmittel dient insbesondere der Führung und/oder dem Halten und/oder dem Anbringen und/oder der Positionierung eines Werkzeuges, das für die Bearbeitung eines Knochens verwendet werden kann. Bei dem Werkzeug kann es sich insbesondere um Schablonen oder Führungen handeln, die ein Knochenbearbeitungswerkzeug, wie zum Beispiel ein Schneidwerkzeug oder einen Knochenbohrer führen. Es kann sich bei dem Werkzeug aber auch um das Knochenbearbeitungswerkzeug an sich, also um einen Bohrer oder das Schneidwerkzeug handeln. Der Bohrer kann insbesondere ein mechanischer Bohrer oder ein Bohrer anderer Art, wie beispielsweise ein Laserbohrer oder ein Wasserstrahlbohrer sein. Entsprechendes gilt auch für das Schneidwerkzeug, das nicht unbedingt mechanisch arbeiten muss, sondern auch das Schneiden mittels zum Beispiel Laserstrahl oder Wasserstrahl vornimmt.

Vorteilhaft umfasst das erfindungsgemäße Bearbeitungshilfsmittel einen ersten Rahmen. Dieser erste Rahmen ist vorzugsweise so gestaltet, dass er an einem Knochen fixierbar ist. Insbesondere kann er eine Knochen-Positionsfixiereinrichtung umfassen, wie zum Beispiel Ösen, durch die passgenau Positionierungsstifte hindurchführbar sind. Entsprechend kann die Knochen-Positionierfixiereinrichtung auch ein Gewinde aufweisen, so dass eine Schraube zum Fixieren des ersten Rahmens hindurchführbar ist. Auch kann die Knochen-Positionsfixiereinrichtung bereits mit Mitteln ausgestattet sein, die direkt in den Knochen einbringbar sind, um den ersten Rahmen an dem Knochen zu fixieren, wie zum Beispiel die vorgenannten Positionierstifte oder Schrauben. Sind 2 oder mehr Fixiermittel, wie Positionierstifte oder Schrauben vorgesehen, so können diese mit ihrer Längsrichtung parallel ausgerichtet sind, Vorzugsweise sind sie schräg zu einander verlaufend, um die Halterung am Knochen zu verbessern.

Vorteilhaft umfasst das erfindungsgemäße Bearbeitungshilfsmittel einen zweiten Rahmen. Dieser dient zum Führen und/oder Halten und/oder Anbringen und/oder Positionieren des Werkzeugs.

Vorteilhaft umfasst das Bearbeitungshilfsmittel weiter eine Justageeinrichtung. Diese ist vorzugsweise so ausgebildet, dass eine räumliche Justage des ersten Rahmens relativ zum zweiten Rahmen ermöglicht wird. Insbesondere ist die relative Lage und/oder Position des ersten Rahmens gegenüber dem zweiten Rahmen durch die Justageeinrichtung einstellbar, insbesondere veränderbar.

Die Justageeinrichtung ist vorzugsweise so gestaltet, dass eine Bewegung des ersten Rahmens relativ zum zweiten Rahmen entlang dreier unterschiedlicher Freiheitsgrade möglich ist. Hierbei handelt es sich insbesondere um eine Drehung um zwei Achsen, die schräg oder orthogonal zueinander sind. Einer der Freiheitsgrade, die zur Justage verwendet werden und deshalb auch als "Justage-Freiheitsgrade" bezeichnet werden, entspricht vorzugsweise einer Translationsbewegung des ersten Rahmens relativ zum zweiten Rahmen. Diese drei Freiheitsgrade dienen insbesondere dazu bei der Knochenbearbeitung die Neigung, den Varus/Valgus und das Resektionsniveau zu bestimmen.

Vorteilhaft ist die Justageeinrichtung so gestaltet, dass eine Relativbewegung zwischen dem ersten und dem zweiten Rahmen (entlang zumindest einem der Justage-Freiheitsgrade, vorzugsweise bezüglich aller Justage-Freiheitsgrade) blockiert ist, wenn eine Kraft auf den ersten Rahmen oder den zweiten Rahmen einwirkt. Dies soll bevorzugt zumindest bis zu einer vorbestimmten Kraft gelten. Diese vorbestimmte Kraft ist vorzugsweise größer als die Kräfte, die bei der Bearbeitung eines Knochens auftreten. Dadurch soll eine beständige und sichere Platzierung des Werkzeugs auch dann gewährleistet werden, wenn beispielsweise Vibrationskräfte aufgrund eines Säge- oder Bohrvorgangs auf das erfindungsgemäße Bearbeitungshilfsmittel wirken.

Die Justage wird vorzugsweise durch ein Justierelement ermöglicht, das die Justageeinrichtung umfasst. Vorzugsweise sind für jeden Freiheitsgrad ein gesondertes Justierelement vorgesehen. Vorzugsweise ist die Kraft, die zur Betätigung des Justierelements erforderlich ist, um eine Relativbewegung des ersten und zweiten Rahmens zu erzielen, geringer als die Kraft, die notwendig ist, um durch Einwirkung auf den ersten oder zweiten Rahmen eine unerwünschte Relativbewegung zu erzielen. Vorzugsweise ist die Justageeinrichtung so gestaltet, dass die Kraft, die eine ungewünschte Relativbewegung aufgrund Einwirkung auf den ersten oder zweiten Rahmen bewirkt, um mehr als 2,5, 10, 20, 50 oder 100 Mal größer ist als die für die Justage erforderliche Kraft. Vorzugsweise ist die Justageeinrichtung selbsthemmend gestaltet, so dass eine Justierbewegung ermöglicht wird, aber einer ungewünschten Verstellung des Justierelements oder der relativen Position zwischen erstem und zweitem Rahmen entgegengewirkt wird. Vorzugsweise ist die Justageeinrichtung so gestaltet, dass über einen Formschluss und/oder mittels Krafteinwirkung und eine durch die Krafteinwirkung bewirkte Verformung und ein dadurch erzielter Formschluss oder mittels Ineinandergreifen nach einem erfolgten Justagevorgang eine Fixierung oder Quasifixierung der Justageeinrichtung erzielt wird. Vorzugweise können elastische Mittel vorgesehen werden, die mittels eines Justierelements leicht verformbar sind, deren elastische Kraft aber so wirkt, dass einer (unerwünschten) Stellung des Justierelements oder einer Dejustage durch Kraftübertragung (über den ersten und/oder zweiten Rahmen) entgegengewirkt wird.

Vorteilhaft kann bei der Justageeinrichtung zwischen einem justierbaren und nichtjustierbaren Zustand gewechselt ist. Dies erfolgt beispielsweise durch einen über elastische Mittel erfolgten Kraftschluss oder über einen Formschluss, wobei beide Arten kombiniert werden können. Insbesondere wird eine pneumatische Steuerung des Wechselvorganges bevorzugt.

Vorzugsweise umfasst die Justageeinrichtung Drehgelenke, insbesondere Kugelgelenke, um eine Bewegung entlang mindestens einem der Freiheitsgrade zu ermöglichen und insbesondere durch ein einziges Drehgelenk die erwünschten, zuvor erwähnten zwei Freiheitsgrade der Drehung zu ermöglichen.

Zusätzlich oder alternativ zu Gelenken kann die Justageeinrichtung eine elastische, insbesondere verbiegbare Strukturen aufweisen, die so gestaltet ist, dass ihre Verbiegung einer Gelenkbewegung entspricht. Insbesondere weist die Strukturen an vorgegebenen Verbiegestellen auf, die insbesondere verjüngt oder aus einem weicheren Material gebildet sind. Die Stellen sind vorzugsweise so gewählt, dass dadurch z.B. über Hebelwirkung Kräfte der Justierelemente verstärkt wirken. Die verbiegbare Struktur ist vorzugsweise einstückig ausgebildet, um eine leichteres Säubern zu ermöglichen und Kosten zu senken. Sie ist vorzugsweise aus Kunststoff.

Vorteilhaft ist an dem zweiten Rahmen ein Bearbeitungsblock fixiert, einstückig damit ausgebildet oder lagefest oder insbesondere lösbar daran anbringbar. Der Bearbeitungsblock dient vorzugsweise insbesondere dem Führen einer Schneidbewegung eines Schneidwerkzeugs oder einer Bohrbewegung eines Bohrwerkzeugs. Alternativ (oder zusätzlich) zu dem vorgenannten Bearbeitungsblock kann auch ein Werkzeug, wie zum Beispiel ein Schneidwerkzeug oder ein Bohrwerkzeug an dem zweiten Rahmen fixiert sein, damit einstückig ausgebildet sein oder lagefest oder insbesondere lösbar an dem zweiten Rahmen anbringbar sein.

Um die Position und/oder Lage des zweiten Rahmens zu überprüfen und bei der Justage optimal einstellen zu können, ist vorzugsweise ein Mittel vorgesehen, das die Erkennung der Lage und/oder Position des zweiten Rahmens erlaubt. Dieses Navigiermittel arbeitet vorzugsweise mit einem Navigationssystem zusammen, dem Lage und Position sowie Oberflächenbeschaffenheit des zu bearbeitenden Knochens bekannt ist. Bei dem Positionsmittel kann es sich beispielsweise um einen Sender handeln. Vorzugsweise ist ein Referenzstern vorgesehen, der insbesondere passive Marker aufweist, die von einem Navigationssystem erfassbar sind. Diese reflektieren beispielsweise elektromagnetische Wellen, wie Licht, Infrarotstrahlen oder UV-Licht oder Ultraschall und so weiter. Der Stern weist vorzugsweise eine derartige geometrische Ausbildung auf, das durch die Erkennung von Markern die Lage des Referenzsterns erfassbar ist. Vorzugsweise ist der Referenzstern lagefixiert bezüglich des zweiten Rahmens an dem Bearbeitungshilfsmittel (zum Beispiel lösbar) anbringbar. Natürlich kann das Navigationsmittel (der Referenzstern) auch einstückig mit dem Bearbeitungshilfsmittel ausgebildet sein. Insbesondere ist er an dem zweiten Rahmen oder dem Werkzeug anbringbar. Vorzugsweise ist das Werkzeug so ausgebildet, dass das Navigationsmittel (Referenzstern) lagefixiert und lösbar mit dem Werkzeug koppelbar ist.

Vorzugsweise umfasst das Navigationsmittel (der Referenzstern) eine (mechanische) Kontaktschnittstelle zum Kontaktieren oder Berühren des zu bearbeitenden oder bereits bearbeiteten Knochens, um dadurch die Lage und/oder Position des Knochens, insbesondere der Knochenschnittfläche oder der Knochenoberfläche zu verifizieren oder abtastend zu erfassen.

Im folgenden werden Ausführungsformen der Erfindung beschrieben. Verschiedene Merkmale dieser Ausführungsformen können untereinander kombiniert werden. Gleiche oder ähnliche Teile tragen gleiche Bezugszeichen soweit nicht anders vermerkt.
Fig. 1 zeigt eine Übersicht einer ersten Ausführungsform der Erfindung.
Fig. 2 zeigt die erste Ausführungsform der Erfindung im Schnitt.
Fig. 3 zeigt eine Detailansicht der ersten Ausführungsform betreffend die translatorische Bewegung.
Fig. 4 zeigt eine Detailansicht der ersten Ausführungsform betreffend die rotatorischen Bewegungen.
Fig. 5a und 5b zeigen eine Vorder- und Rückansicht einer zweiten Ausführungsform der Erfindung.
Fig. 6 zeigt eine Detailansicht der zweiten Ausführungsform der Erfindung mit einer Blattfederlösung.
Fig. 7 zeigt eine alternative Lösung der zweiten Ausführungsform mit einer einstückigen verformbaren Struktur.
Fig. 8 zeigt eine dritte Ausführungsform der Erfindung von einer Seite.
Fig. 9 zeigt die dritte Ausführungsform der Erfindung von einer anderen Seite.
Fig. 10 zeigt eine vierte Ausführungsform der Erfindung mit schräg gestellter Platte.
Fig. 11 zeigt die vierte Ausführungsform der Erfindung mit paralleler Platte.

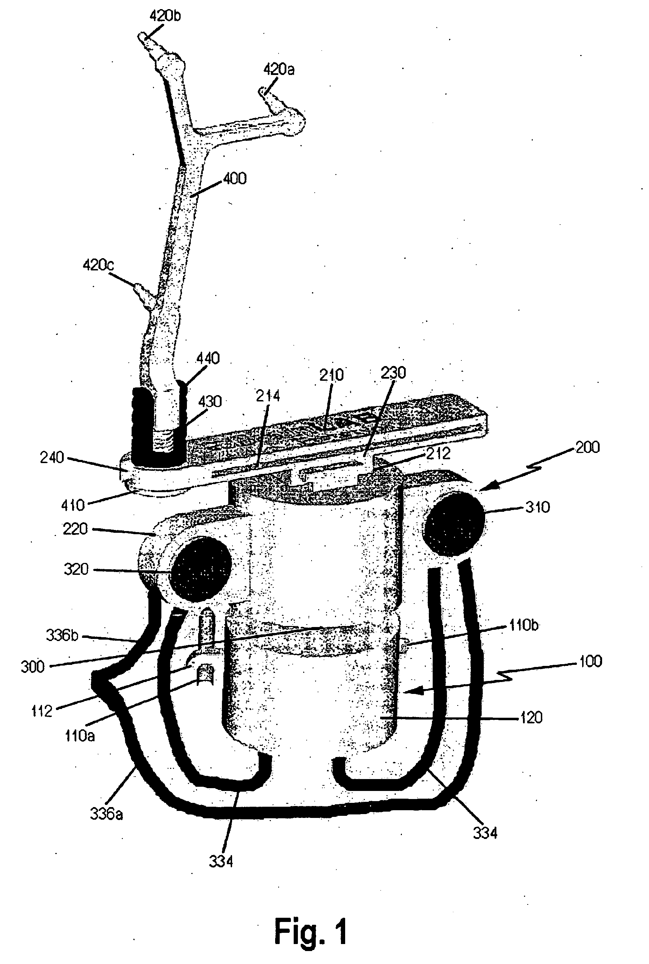

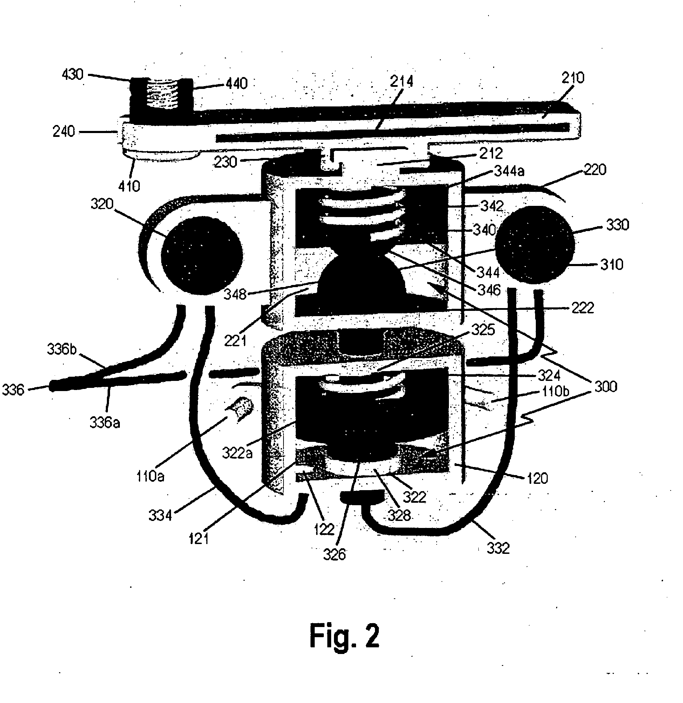

Fig. 1 zeigt eine erste Ausführungsform, die zum Lösen einer Arretierung oder Blockierung des Justageeinrichtung 300 pneumatische Kräfte (alternativ auch zum Beispiel hydraulische Kräfte) nutzt. Das Gehäuse 220 ist dem zweiten Rahmen zuzurechnen. Innerhalb des Gehäuses 220 befindet sich ein Teil der Justiereinrichtung. Das Gehäuse 120 ist dem ersten Rahmen zuzurechnen. Innerhalb dieses Gehäuses befindet sich ein weiterer Teil der Justiereinrichtung. Verbunden ist das erste Gehäuse 120 und das zweite Gehäuse 220 über die Justiereinrichtung 300, die nur leicht zwischen dem Gehäuse 120 und dem Gehäuse 220 in Fig. 1 zu erkennen ist. Schläuche 332 und 334 für ein Fluid, insbesondere ein Gas, insbesondere Luft sind vorgesehen, die steuerbar über Ventile 310 und 320 das Fluid zur pneumatischen (oder hydraulischen) Steuerung der Justiereinrichtung in die Gehäuse 120 und 220 einführt, wie weiter unten im Zusammenhang mit Fig. 2 näher erläutert wird. 230 stellt eine Schiene dar, die eine mechanische Schnittstelle zu dem Werkzeug 210 bildet. Das Werkzeug 210 und die Schnittstelle 230 sind vorzugsweise so ausgebildet, dass eine feste Position, insbesondere die Entfernung zum zu bearbeitenden Knochen, einstellbar ist. Beispielsweise ist hierzu entlang des Schlittens 212 des Werkzeugs 210, das entlang der Schiene 230 gleiten kann, eine Verrasterung vorgesehen, die in ein nicht dargestelltes Rastelement außerhalb der Schiene 212 eingreifen kann, um so das Werkzeug 210 einrastend in Richtung auf einen Knochen vorwärtsschieben zu können, bis die gewünschte, insbesondere sehr nahe Position oder Kontaktposition bezüglich des Knochens erreicht wurde. Vorzugsweise ist die Verrasterung so ausgebildet, dass ein Zurückziehen des Werkzeugs nicht mehr möglich ist, so dass das Werkzeug 210 erst nach Lösen des gesamten Bearbeitungshilfsmittels von dem Knochen abziehbar ist, indem das Werkzeug 210 weiter in dieselbe Richtung vorangeschoben wird.

Verbunden ist das erste Gehäuse 120 (erster Rahmen 100) mit dem Knochen über Stifte 110a und 110b, die vorzugsweise passgenau oder in Presspassung durch Ösen 112 geführt werden, die vorzugsweise ortsfest mit dem Gehäuse 120 verbunden sind. Alternativ kann dadurch beispielsweise auch anstelle der Stifte eine Schraube und anstelle der Ösen eine Bohrung mit zu der Schraube passendem Innengewinde vorgesehen werden. Auch andere Befestigungsverfahren, zum Beispiel mittels Kleben oder Verhakungen sind denkbar.

Bei dem Werkzeug 210 handelt es sich um einen Schneidblock mit Schlitz 214, durch das ein Schneidwerkzeug, insbesondere ein Sägeblatt geführt werden kann.

An dem Werkzeug 210 ist ein Referenzstern 400 lösbar befestigt. Der Referenzstern 400 weist unten eine Kontaktplatte 410 auf, die im gelösten Zustand des Referenzsterns mit der Schnittfläche eines Knochens in Kontakt gebracht werden kann, insbesondere plan aufgelegt werden kann, um Lage und Position der Schnittfläche zu vermessen. Der Referenzstern 400 weist drei passive Marker 420a, 420b und 420c auf, die vorzugsweise eine Ebene aufspannen. Die Verbindung zwischen dem Referenstern 400 und dem Werkzeug 410 ist vorzugsweise durch eine Schnappverbindung ausgebildet, die ein elastisches Mittel, insbesondere eine Feder 430 aufweist, die um eine nicht sichtbare Achse geführt ist. Die Feder 430 drückt eine Hülse 440 in eine komplementär ausgebildete Ausnehmung des Werkzeugs 210 nach unten, so dass sich ein Formschluss ergibt. Durch Anheben der Hülse 440 gegen die Federkraft 430 wird der Formschluss gelöst und die nicht gezeigte Achse, die von der Feder 430 axial umgeben wird, kann durch den Schlitz 240 im Werkzeug 210 seitlich herausgeführt werden.

Fig. 2 zeigt den Aufbau der Justageeinrichtung 300. Gleiche Bezugszeichen in Fig. 2 bezeichnen die gleichen bereits in Verbindung mit Fig. 1 beschriebenen Teile.

Druckluft wird über einen Schlauch 336 der Justiereinrichtung 300 zugeführt. Dazu wird der Schlauch 336 in zwei Schläuche 336b und 336a aufgespaltet. Der Schlauch 336b führt die Druckluft zu einem Ventil 320, das von außen betätigbar ist, beispielsweise durch Drücken auf den mit "TRANS" bezeichneten Knopf. Wird das Ventil 320 geöffnet, so gelangt die Druckluft weiter über den Schlauch 334 in das innere des Gehäuses 120, und zwar durch die Öffnung 122. Die Druckluft im inneren des Gehäuses 120 bewirkt ein Anheben der Dichtscheibe 322, die luftdicht entlang der Innenwand des Gehäuses 120 gleiten kann und Luft nicht nach oben passieren lässt. Die Dichtscheibe 322 wird durch die Feder 324 nach unten gedrückt und wirkt über einen Pressring 326 auf eine verformbare Konusscheibe 328. Die Feder 324 umgibt axial eine Achse 325, die zumindest im Bereich der Konusscheibe 328 radial um die Achse 325 verlaufende Rillen oder Rastervertiefungen aufweist, in die die Konusscheibe eingreift, wenn diese durch Druckausüben des Pressrings 326 verformt wird. Die Dichtescheibe 322, der Pressring 326 und die Konusscheibe umgeben die Achse 325 axial und sind entlang dieser beweglich.

Wird Druckluft in die Kammer des Gehäuses 120 durch die Öffnung 122 eingeführt, so wird die Dichtscheibe 322 angehoben und der Pressring 326 drückt nicht mehr auf die Konusscheibe 328, so dass diese nicht mehr verformt ist und auch nicht mehr in die Verrasterung bzw. Rillen der Achse 325 eingreift. Die Achse 325 ist also dann frei relativ des Gehäuses 120 zum Beispiel durch einen Chirurgen beweglich. Somit kann durch Druckluftzufuhr über das Ventil 320 eine Translationsbewegung des Gehäuses 220 relativ zum Gehäuse 120 ermöglicht werden.

Das Gehäuse 220 weist eine sphärische Öffnung 222 auf, in der eine Kugel 330 steckt, die fest mit der Achse 325 verbunden ist. Die sphärische Öffnung 222 weist vorzugsweise einen kleineren Durchmesser auf, als die Kugel 330. Insbesondere liegt sie unterhalb des Meridians der Kugel 330. Das Gehäuse 220 ist mit der Kugel 330 über Kraftschluss und/oder Formschluss verbunden. Dies wird mittels einer Feder 340 bewirkt, die um eine Achse 342 axial gelagert ist. Diese Achse 342 ist innerhalb des Gehäuses 220 einstückig mit dem gleitfähigen Element 344 verbunden, das eine untere Kammer 221 innerhalb des Gehäuses nach oben ablichtet und einen Anschlag 344a aufweist, so dass eine Spitze 346 des Elements nur um einen vorbestimmten Abstand von der Kugel 348 entfernt werden kann.

Dieses Element 344 gleitet ähnlich wie die Dichtscheibe 322 luftdicht abschließend entlang der Innenwand des Gehäuses 220, das ebenso wie das Gehäuse 120 vorzugsweise zylinderförmig ausgebildet ist. Am unteren Ende des Gleitelements 344 ist vorzugsweise die kegelstumpfarrig nach unten in Richtung auf die Kugel sich verjüngende Eingreifspitze 346 vorgesehen, die an ihrem unteren Ende eine zylindrische Vertiefung 348 aufweist.

Die Spitzen des Elements 346 werden durch die Federkraft der Feder 340 gegen die Oberfläche der Kugel 330 gepresst. Vorzugsweise ist die Oberfläche der Kugel 330 verformbar, um zusätzlich zu einem Kraftschluss auch noch einen Formschluss zu erzielen.

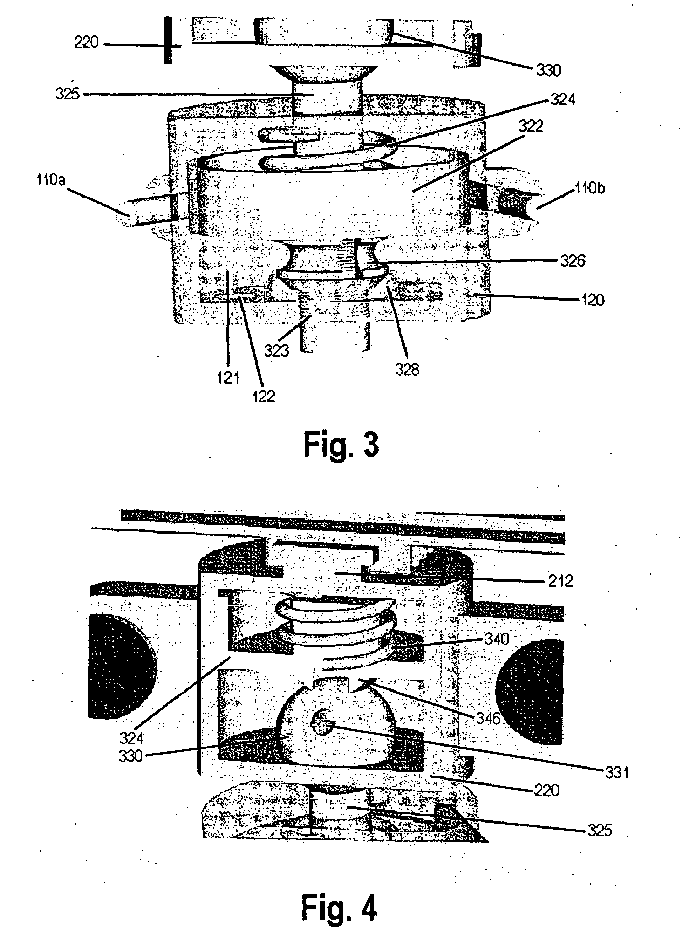

Wird Druckluft über die Leitung 336 zu dem Ventil 310 zugeführt und das Ventil 310 geöffnet, so gelangt diese Luft über den Schlauch 332 in die hohl ausgebildete Achse 325. Die Druckluft wird durch einen vorzugsweise zylindrischen Hohlraum durch das Innere der Achse 325 geführt und gelangt durch eine in Figur 4 gezeigte Öffnung 331 in der Kugel 330 in den Innenraum des Gehäuses 220, genauer in den unteren Innenraum 221, über dem das Element 344 liegt. Die Druckluft bewirkt dann eine Kraft auf das Element 344, die der Kraft der Feder 340 entgegenwirkt. Auf diese Art und Weise wird die Spitze 346 von der Kugel entfernt und das Gehäuse 220 ist nicht mehr zwischen der Spitze 346 und der sphärische Öffnung 222 eingeklemmt, so dass das Gehäuse frei um die Kugel 330 drehbar ist (zwei Freiheitsgrade der Rotation).

In den mit Druckluft befüllbaren Kammern 221 und 121 der Gehäuse 220 und 120, die durch die Elemente 344 und 322 jeweilig nach oben abgeschlossen sind, kann ein Druckabbau entweder über die Schläuche erfolgen, die auch die Druckluft zuführen oder es sind in den Gehäusen 120 und 220 jeweils Öffnungen vorgesehen, die ein langsames Entweichen der Druckluft erlauben, so dass ein automatischer Druckabbau erfolgt, wenn die Ventile 320 und 310 geschlossen werden. Dieser Druckabbau hat dann eine Arretierung der Justageeinrichtung zur Folge. Die Spitze 346 drückt wieder auf die Kugel 330 und die Konusscheibe 328 wird wieder zusammengepresst, so dass sie in die Rillen der Achse 325 eingreift.

Fig. 3 zeigt den Teil der Justageeinrichtung genauer, der für die Translationsbewegung verantwortlich ist. Der Pressring 326 ist offen so gezeichnet, dass die Rillenstruktur 323 zu erkennen ist, die entlang der Achse 325 verläuft. Drückt der ebenfalls am unteren Ende konisch ausgebildete Pressring 326 auf die an ihrem unteren Ende ringförmig ausgebildete Konusscheibe 328, so wird diese aufgrund ihrer elastischen Ausbildung zusammengedrückt, so dass ihr unteres, ringförmiges Ende zwischen die Rillen 323 eingepresst wird, wodurch eine translatorische Bewegung der Achse 325 relativ zum Gehäuse 120 verhindert wird.

Durch Einströmen von Druckluft durch die Öffnung 122 in die unterhalb der Dichtscheibe 322 ausgebildete Kammer 121 wird die Dichtscheibe 322 angehoben, so dass die Konusscheibe 328 nicht mehr gepresst wird und nicht mehr verformt ist, da der Pressring 326, der mit der Dichtscheibe 322 verbunden ist, durch die Druckluft, die auf den Dichtring 322 wirkt, nach oben angehoben wird. Dadurch greift der konische Ring 328 nicht mehr in die Rillen 321 ein und die Achse 325 ist frei relativ zum Gehäuse 120 verschiebbar.

Die Fig. 4 zeigt den rotatorischen Teil der Justageeinrichtung vergrößert. Gleiche Bezugszeichen bezeichnen wiederum gleiche Teile wie sie bereits im Zusammenhang mit Fig. 1 und 3 erläutert wurden. Zusätzlich zu den bereits erläuterten Teilen ist in Fig. 4 noch eine Öffnung 331 in der Kugel 330 sichtbar, durch die die Druckluft austritt, die über den Schlauch 332 dem Hohlraum der Achse 325 zugeführt wird.

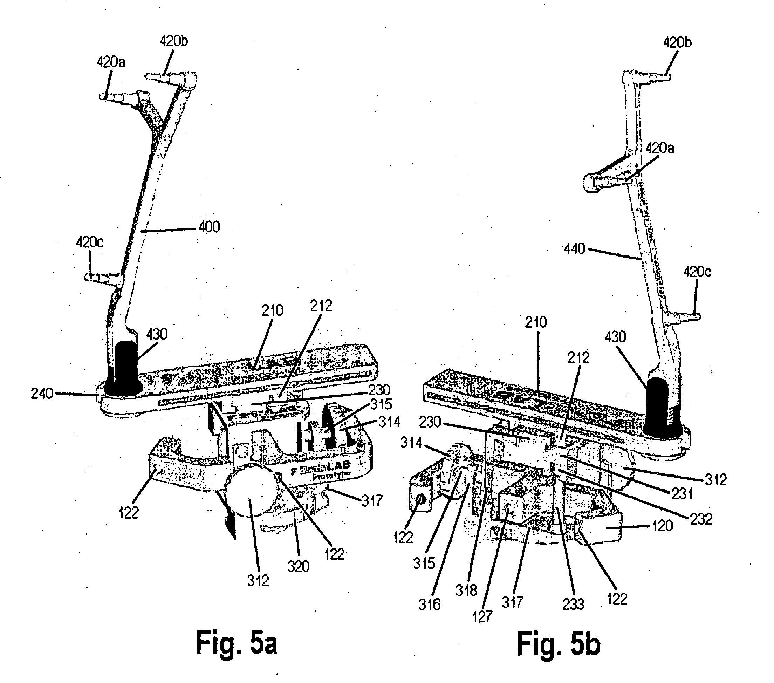

Fig. 5a und 5b zeigen eine zweite Ausführungsform der vorliegenden Erfindung. Diese arbeitet im Gegensatz zur ersten Ausführungsform nicht pneumatisch, sondern mechanisch, wobei elastisch ausgebildete Elemente dazu dienen, die eingestellte relative Lage zwischen dem ersten und zweiten Rahmen zu fixieren, und insbesondere gegenüber Erschütterungen zu sichern.

Der erste Rahmen 120 weist Öffnungen 122 auf, durch die Positionierstifte geführt werden können, um den ersten Rahmen mit dem Knochen zu verbinden.

Die linke Figur, die Fig. 5a, weist mit der Seite zum Betrachter, die ein' Chirurg bei der Anwendung des Bearbeitungshilfsmittels sieht. Die rechte Seite der Fig. 5, die Fig. 5b, weist mit der Seite zum Betrachter, die dem zu bearbeitenden Knochen zugewandt ist.

Die Rädchen 312 und 314 erlauben die Drehung einer Gewindestange 315, die nur bezüglich des Rädchens 314 gezeigt ist. Die Gewindestange 315 verläuft in einem dazu komplementären Gewinde der Gewindestangenhalterung 316. Diese Gewindestangenhalterung 316 ist ortsfest mit einer unteren Rahmenstruktur 317 verbunden. An dieser unteren Rahmenstruktur 317 ist ein verbiegbares Element, insbesondere eine Blattfeder 318 angebracht. Durch Drehen des Einstellrings 314 wird die Gewindestange 315 hin oder weg von dem elastischen Element (Blattfeder) 318 geführt. Wird sie hingeführt, so kann dadurch das elastische Element, insbesondere die Blattfeder 318 verbogen werden. Während das eine Ende der Blattfeder 318 mit der unteren Rahmenstruktur 317 verbunden ist, ist das andere Ende ortsfest über eine obere Rahmenstruktur 231 mit der Schiene 230 verbunden, die als Schnittstelle zu dem Werkzeug 210 dient. Durch Krümmen des elastischen Elements aufgrund der Krafteinwirkung der Gewindestange 315 wird der Abstand zwischen der unteren Rahmenstruktur 317 und der oberen Rahmenstruktur 231 im Bereich der Blattfeder 318 verkürzt. Die obere Rahmenstruktur 231 ist mit einem Kugelgelenk 232 so verbunden, dass es um dieses rotieren kann, wenn sich der vorgenannte Abstand verkürzt. Diese Rotation wird also durch die Verkürzung der Blattfeder 318 bewirkt, so dass sich ein Verkippen der Schnittstelle, bzw. der Schiene 230 und damit des Werkzeugs 210 um den durch das Kugelgelenk 232 festgelegten Punkt ergibt. Das Kugelgelenk 232 befindet sich in einem durch die Stange 233 fixierten Abstand zu der unteren Rahmenstruktur 317.

Das andere Einstellrädchen 312 wirkt in der gleichen Weise wie das Einstellrädchen 314 auf eine weitere Blattfeder, die analog angeordnet ist. Die Gewindestange, die dem Einstellrädchen 312 zugeordnet ist, weist vorzugsweise ebenso wie die Gewindestange 315 in Richtung auf das Kugelgelenk 232 bzw. die Halterung 233 für das Kugelgelenk. Die Achsen der Gewindestangen stehen vorzugsweise in einem rechten Winkel oder zumindest schräg zueinander. Auf diese Art und Weise wird bei Krafteinwirkung der Gewindestange, die dem Einstellrädchen 312 zugeordnet ist, eine Verkippung des Werkzeugs 210 um das Kugelgelenk 232 bewirkt, die unabhängig, insbesondere rechtwinklig zu der Verkippung ist, die durch das andere Einstellrädchen 314 bewirkbar ist, wodurch die zwei gewünschten Rotations-Freiheitsgrade der Justage erzielt werden.

Während die Rädchen 312 und 314 eine Rotationsbewegung um zwei zueinander senkrecht stehende Achsen bewirken, erlaubt das dritte Einstellrädchen 320 eine translatorische Bewegung. Hierzu ist in dem ersten Rahmen 120 ein Gewinde ausgebildet. Eine weitere Gewindestange, die dem Einstellrädchen 320 zugeordnet ist, das heißt mit diesem drehfest verbunden ist, greift in das Gewinde des ersten Rahmens 120 ein. Durch Drehen des Rädchens 320 verändert sich also der Abstand des Rädchens 320 zu dem ersten Rahmen 120. Das Rädchen 320 ist schließlich mit der unteren Rahmenstruktur 317 so verbunden, dass sich bei Abstandsänderung zwischen dem Rädchen 320 und dem ersten Rahmen 120 auch eine entsprechende Abstandsänderung zwischen der unteren Rahmenstruktur 317 und dem ersten Rahmen 120 einstellt, da die untere Rahmenstruktur so ausgebildet ist, dass sie von dem Rädchen 320 mitgenommen wird.

Alternativ zur vorher beschriebenen Realisierung der translataischen Einstellbarkeit kann auch das Rädchen 320 ein Innengewinde aufweisen und um die zugeordnete Gewindestange drehbar sein. In diesem Fall ist dann die Gewindestange drehfest am ersten Rahmen 120 angebracht oder einstückig mit diesem ausgebildet. So kann auch bei dieser Lösung durch drehen des Rädchens 320 der Abstand des Rädchens 320 zum ersten Rahmen 120 verändert werden. Diese alternative Realisierung der translataischen Justage ist auf alle hier beschriebenen Ausführungsformen anwendbar.

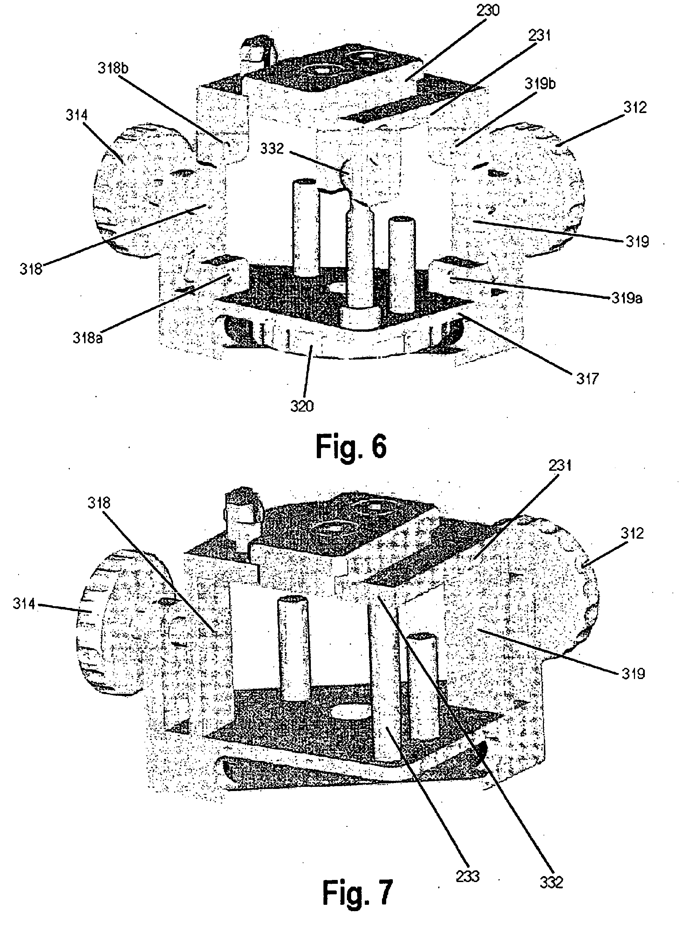

Um eine Verkippung entlang der Drehachsen zu ermöglichen, sind die Blattfedern mit der oberen und unteren Rahmenstruktur vorzugsweise über ein Gelenk 318a und 318b verbunden, wie aus Fig. 6 ersichtlich ist.

Fig. 6 zeigt auch die dem Einstellrädchens 312 zugeordnete Blattfeder 319, die ebenfalls über zwei Gelenke 319a und 319b mit der oberen und unteren Rahmenstruktur 317 und 231 verbunden ist.

Fig. 7 zeigt eine andere Variante der zweiten Ausführungsform, bei der keine Blattfedern vorgesehen sind, sondern die gesamte Struktur bis auf die Einstellrädchens mit den zugeordneten Gewindestangen einstückig ausgebildet ist. Diejenigen Teile, die sich verformen und bewegen müssen, sind vorzugsweise dünnwandig ausgebildet. Insbesondere ist derjenige Streifen (bevorzugt Kunststoffstreifen), der an derselben Stelle wie die Blattfeder 318 angeordnet ist und deshalb in Fig. 7 auch mit demselben Bezugszeichen versehen wurde, dünnwandig ausgebildet. Entsprechendes gilt für die dünne Wand 319, die dem Rädchen 312 zugeordnet ist. Durch Krafteinwirkung der den Rädchen 312 und 314 jeweils zugeordneten Gewindestangen auf die dünnen Wände 318 und 319 werden diese verbogen, so dass sich eine Rotationsbewegung um den Verbindungspunkt 332 zwischen der oberen Rahmenstruktur 231 und der Stange 233 ergibt. Diese Stange 233 ist vorzugsweise ebenfalls dünn ausgebildet, um ein Verbiegen der Stange zu ermöglichen. Auf diese Art und Weise kann ebenfalls eine Drehbewegung um zwei einander senkrecht stehende Achsen erzielt werden und die Anzahl der Bauelemente kann verringert werden. Dies ist insbesondere vorteilhaft, um eine Reinigung des Bearbeitungshilfsmittels zu erleichtern.

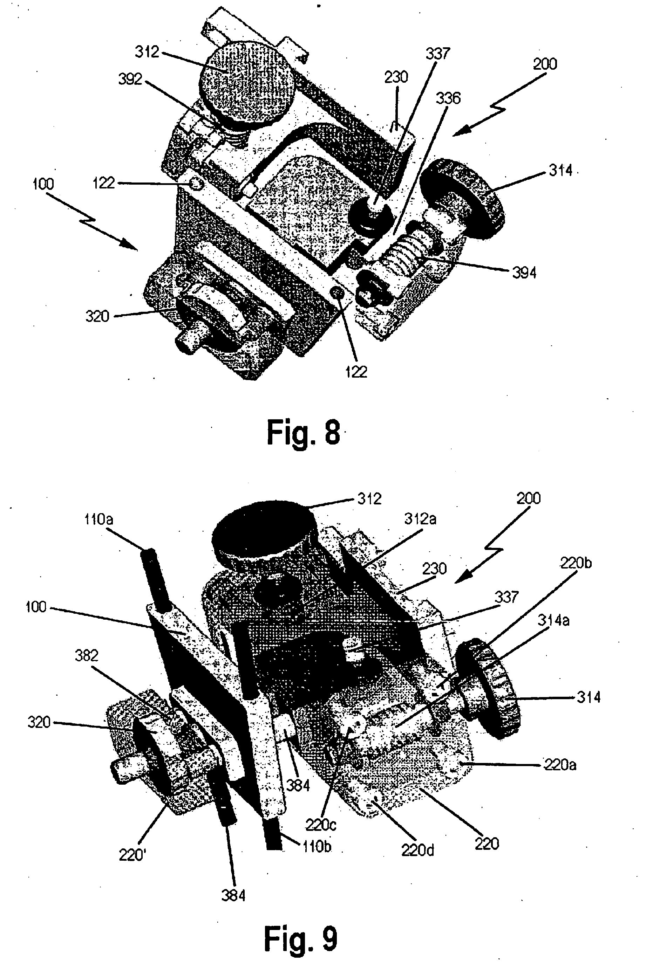

Fig. 8 zeigt eine dritte Ausführungsform der vorliegenden Erfindung, die zur Verstellung um die Rotationsachsen ein selbsthemmendes Getriebe, insbesondere ein Schneckengetriebe verwendet. Das Schneckengetriebe 392 wird durch das Rädchen 312 betätigt und das Schneckengetriebe 394 durch das Rädchen 314. Die Schneckengetriebe 392 und 394 sind über ein nicht gezeigtes Lager gelagert, das ortsfest mit dem zweiten Rahmen 200 verbunden ist. Die Schneckengetriebe 394 und 392 kämmen mit jeweils einem Zahnrad, wobei nur das Zahnrad 336 gezeigt ist, das mit dem Schneckengetriebe 394 kämmt. Die Achsen der Zahnräder sind im 90°-Winkel zu den Achsen der Schneckengetriebe angeordnet. Die Zahnräder bewirken eine Drehung um eine Drehachse 337 und um eine weitere nicht gezeigte Drehachse, die ebenfalls senkrecht zu der Achse des jeweilig zugeordneten Schneckengetriebes verläuft. Hierdurch werden wiederum die zwei gewünschten Rotationsfreiheitsgrade der Justage erzielt.

Um ein besonders leicht reinigbares Bearbeitungsmittel zu erzielen, sind die für die Rotation verantwortlichen Justagemittel wie die Schneckengetriebe, die zugeordneten Zahnräder und Drehachsen innerhalb eines Gehäuses 220 angeordnet, das mit Schrauben 220a - 220d an einer nicht gezeigten Rahmenstruktur, die einstückig mit der Schnittstelle zu dem Werkzeug verbunden ist, festgeschraubt.

Eine translatorische Bewegung wird mit Hilfe des Rädchens 320 erzielt, das, wie auch bei der vorherigen Ausführungsform, einstückig mit einer Gewindestange ausgebildet ist, die sich mit dem Rädchen dreht und in einem komplementären Gewinde verläuft. Dieses komplementäre Gewinde ist in dem ersten Rahmen 100 ausgebildet. In dem ersten Rahmen 100 befinden sich auch wiederum Öffnungen 122, durch die Positionierstifte 110a und 110b führbar sind (siehe Fig. 9). Ein weiteres Gehäuse 220' umgibt das Rädchen 320 bis auf einen Schlitz, durch den das Rädchen 320 herausschaut, um es bedienen zu können. Durch Drehen des Rädchens 320 wird der Abstand zwischen dem Rädchen 320 und dem ersten Rahmen 100 eingestellt. Das Rädchen 320 ist über zwei Stangen 382 und 384 mit der Schnittstelle 230 zu dem Werkzeug ortsfest über die nicht gezeigte Rahmenstruktur verbunden. Durch Veränderung des Abstandes des Rädchens 320 zu dem ersten Rahmen 100 ändert sich über die Stangen 382 und 384 auch der Abstand zwischen dem ersten Rahmen 100 und dem zweiten Rahmen 200. Auf diese Art und Weise kann durch das Rädchen 320 eine translatorische Justagebewegung erzielt werden.

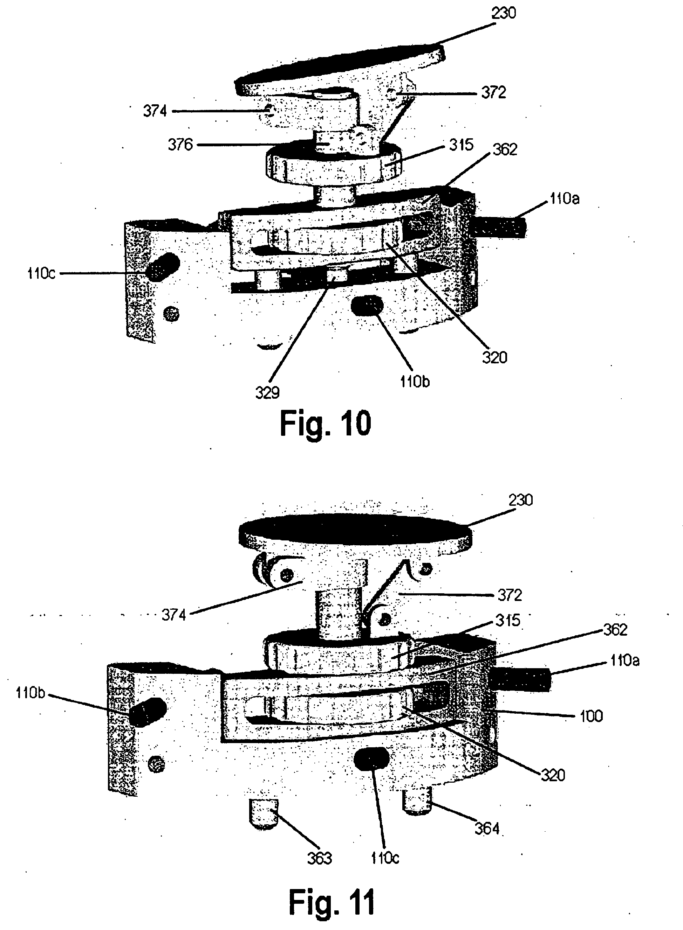

Fig. 10 und Fig. 11 zeigen eine vierte Ausführungsform der Erfindung. Bei dieser genügt ein Rädchen 315, um eine Rotation der Schnittstelle 230 zu dem Werkzeug um zwei Rotationsachsen zu bewirken, um die gewünschten zwei Rotations-Freiheitsgrade zu erzielen. Das Rädchen 315 ist über eine Gelenkverbindung 372 mit einer Seite der Schnittstelle 230 verbunden. Die andere Seite, vorzugsweise eine gegenüberliegende Seite der vorzugsweise plattenförmig (insbesondere als Rundplatte) ausgebildeten Schnittstelle 230 ist ebenfalls über eine Gelenkverbindung 374 mit einer Gewindestange 376 verbunden. Während das Rädchen 315 über die Gelenkverbindung 372 also mit einer Seite der Platte 230 verbunden ist, ist die Gewindestange 376 über die Gelenkverbindung 374 mit der anderen, gegenüberliegenden Seite der Platte 230 verbunden. Das Rädchen 315 hat ein Innengewinde, das mit dem Außengewinde der Gewindestange 376 in Eingriff ist. Durch Drehen des Rädchens 315 wandert das Rädchen 315 also entlang der Stange 376. Wird somit das Rädchen 315 auf die Platte 230 zu bewegt, so kippt diese über die Gelenkverbindung 372 die Platte 230 in immer stärkerem Maße. Gleichzeitig dreht sich auch die Platte 230 mit dem Rädchen 315 mit, da die Gelenkverbindung 374 drehbar bezüglich der Gewindestange 376 gelagert ist, so dass sie um die Achse der Gewindestange 376 drehbar ist. Auf diese Art und Weise dreht sich die Platte 230 bei Drehen des Rädchens 315 ebenfalls um eine Drehachse, die mit der Achse der Gewindestange 376 übereinstimmt. Somit können abhängig von der Feinheit der Gewindehöhe der Gewindestange 376 verschiedenste Kipplagen der Platte bzw. Schnittstelle 230 um zwei zueinander senkrecht stehende Rotationsachsen eingestellt werden.

Während Fig. 10 die gekippte Platte 230 zeigt, zeigt Fig. 11 die ebene Platte 230. Die translatorische Bewegung ist wiederum über das Rädchen 320 erreichbar. Während die Platte 230 dem zweiten Rahmen zuzuordnen ist, ist der erste Rahmen wiederum mit dem Bezugszeichen 100 versehen. Der erste Rahmen 100 weist Positionierstifte 110a, 110b und 110c auf, die in einen Knochen getrieben werden können. Die Justiereinrichtung dieser Ausführungsform weist auch eine vermittelnde Rahmenstruktur 362 auf, die der translatorischen Bewegung dient. Diese Rahmenstruktur 362 ist durch Stifte 363 und 364 in dazu komplementären zylindrischen Bohrungen im Rahmen 100 geführt. Der Abstand des vermittelnden Rahmens 362 zu dem ersten Rahmen 100 ist durch Drehen des Rädchens 320 einstellbar. Das Rädchen 320 ist drehfest mit einer Gewindestange 329 verbunde, die im Eingriff mit einem dazu komplementären Gewinde in dem ersten Rahmen 100 ist. Durch Drehen des Rädchens 320 ändert sich somit der Abstand des Rädchens 320 zu dem ersten Rahmen 300. Das Rädchen 320 nimmt dabei die vermittelnde Rahmenstruktur 362 mit, die mit Hilfe der Stifte 362 und 364 verdrehfest, aber translatorisch bewegbar im ersten Rahmen 100 gelagert ist. Somit wird dann die translatorische Bewegung des vermittelnden Rahmens 362 relativ zum ersten Rahmen bewirkt. Die oben beschriebene Gewindestange 376 ist wiederum fest mit dem vermittelnden Rahmen 362 verbunden, so dass die translatorische Bewegung des vermittelnden Rahmens 362 auf die Platte 230 übertragen wird.

## Patentansprüche

1. Justierbares Bearbeitungshilfsmittel für die Führung, das Halten, das Anbringen und/oder die Positionierung eines Werkzeugs für die Bearbeitung eines Knochens, insbesondere zur Führung eines Schneidwerkzeuges und zum Positionieren eines Schneidblockes für eine Knochen-Resektion ;
mit einem ersten Rahmen (100), der an einem Knochen fixierbar ist;
mit einem zweiten Rahmen (200) zum Führen, Halten, Anbringen und/oder Positionieren des Werkzeugs;
mit einer Justageeinrichtung (300), die eine räumliche Justage des ersten Rahmens (100) relativ zum zweiten Rahmen (200) erlaubt; **dadurch gekennzeichnet, dass** das justierbare Bearbeitungshilfsmittel einen Referenzstern enthält, der
a) an dem zweiten Rahmen lösbar anbringbar ist, um die Lage und/oder Position des zweiten Rahmens zu erkennen, und
b) der eine Kontaktschnittstelle (410) zum Kontaktieren des zu berarbeitenden oder bearbeiteten Knochens aufweist, um im gelösten Zustand die Lage und/oder Position des Knochens durch Berührung abtastend zu erfassen.

2. Bearbeitungshilfsmittel nach Anspruch 1, bei welchem die Justageeinrichtung drei Justage-Freiheitsgrade aufweist.

3. Bearbeitungshilfsmittel nach Anspruch 1 oder 2, bei welchem die Justageeinrichtung für die Drehung um zwei Achsen ausgebildet ist.

4. Bearbeitungshilfsmittel nach einem der Ansprüche 1 bis 3, bei welchem die Justageeinrichtung für eine Translationsbewegung ausgebildet ist.

5. Bearbeitungshilfsmittel nach einem der Ansprüche 1 bis 4, bei welchem die Justageeinrichtung (300) so gestaltet ist, dass eine Relativ-Bewegung zwischen dem ersten und dem zweiten Rahmen entlang zumindest einem der Justage-Freiheitsgrade blockiert ist, wenn eine Kraft auf den ersten oder zweiten Rahmen wirkt, aber ermöglicht ist, wenn ein Justierelement der Justageeinrichtung verwendet wird.

6. Bearbeitungshilfsmittel nach einem der Ansprüche 1 bis 5, bei welchem die Justageeinrichtung zwischen einem justierbaren und nicht-justierbaren Zustand insbesondere mittels pneumatischer oder hydraulischer Kräfte wechselbar gestaltet ist.

7. Bearbeitungshilfsmittel nach einem der Ansprüche 1 bis 6, bei welchem die Justageeinrichtung Drehgelenke aufweist, die ein Bewegung entlang mindestens einem Freiheitsgrad erlauben.

8. Bearbeitungshilfsmittel nach einem der Ansprüche 1 bis 7, bei welchem der zweite Rahmen so gestaltet ist, dass ein Bearbeitungsblock (210) zum Führen einer Schneid- oder Bohrbewegung an dem zweiten Rahmen fixiert ist, einstückig damit ausgebildet ist oder lagefest und insbesondere lösbar anbringbar ist.

9. Bearbeitungshilfsmittel nach einem der vorhergehenden Ansprüche, bei welchem das Werkzeug ein Schneidblock, eine Bohrschablone, ein Schneidwerkzeug oder ein Bohrer ist.

10. Bearbeitungsmittel nach einem der vorhergehenden Ansprüche, bei welchem die Justageeinrichtung eine verbiegbare, insbesondere elastische, insbesondere einstückige Struktur aufweist, deren Verbiegbarkeit so gestaltet ist, dass die Verbiegung der Struktur durch Justierelemente vorgegebenen Justage-Freiheitsgraden entspricht.

## Claims

1. An adjustable treatment aid for guiding, holding, attaching and/or positioning a tool for treating a bone, in particular for guiding an incision tool and for positioning an incision block for osteoresection, comprising:
a first frame (100) which can be fixed to a bone;
a second frame (200) for guiding, holding, attaching and/or positioning the tool;
an adjusting device (300) which allows the first frame (100) to be spatially adjusted relative to the second frame (200);
**characterised in that** the adjustable treatment aid includes a reference star which:
a) can be detachably attached to the second frame, in order to identify the position of the second frame; and
b) comprises a contact interface (410) for contacting the bone which is to be treated or has been treated in order, when detached, to detect the position of the bone by scanning by contact.

2. The treatment aid according to claim 1, wherein the adjusting device exhibits three adjustment degrees of freedom.

3. The treatment aid according to claim 1 or 2, wherein the adjusting device is formed for rotation about two axes.

4. The treatment aid according to any one of claims 1 to 3, wherein the adjusting device is formed for a translational movement.

5. The treatment aid according to any one of claims 1 to 4, wherein the adjusting device (300) is designed such that a relative movement between the first and second frame along at least one of the adjustment degrees of freedom is blocked when a force acts on the first or second frame, but is enabled when an adjusting element of the adjusting device is used.

6. The treatment aid according to any one of claims 1 to 5, wherein the adjusting device is designed such that it can alternate between an adjustable and a non-adjustable state, in particular by means of pneumatic or hydraulic forces.

7. The treatment aid according to any one of claims 1 to 6, wherein the adjusting device comprises rotary joints which allow movement along at least one degree of freedom.

8. The treatment aid according to any one of claims 1 to 7, wherein the second frame is designed such that a treatment block (210) for guiding an incision or drilling movement is fixed to the second frame, formed as one piece with the second frame or can be attached, positionally fixed and in particular detachably, to the second frame.

9. The treatment aid according to any one of the preceding claims, wherein the tool is an incision block, a drilling template, an incision tool or a drill.

10. The treatment aid according to any one of the preceding claims, wherein the adjusting device is a bending, in particular elastic, in particular one-piece structure which is designed to be able to bend such that the bending of the structure by adjusting elements corresponds to predetermined adjustment degrees of freedom.

## Revendications

1. Support d'usinage réglable destiné à guider, maintenir, poser et/ou positionner un outil pour l'usinage d'un os, en particulier destiné à guider un outil de coupe et à positionner un bloc de coupe pour une résection osseuse ;
avec un premier bâti (100), qui est apte à être fixé contre un os ;
avec un deuxième bâti (200) destiné à guider, maintenir, poser et/ou positionner l'outil ;
avec un dispositif d'ajustement (300), qui permet un ajustement dans l'espace du premier bâti (100) par rapport au deuxième bâti (200) ;
**caractérisé en ce que** le support d'usinage réglable comporte une étoile de référence qui
a) est apte à être montée de manière amovible sur le deuxième bâti, afin de détecter l'emplacement et/ou la position du deuxième bâti, et
b) comporte une interface de contact (410) destinée à être mise en contact sur l'os à usiner ou usiné, afin de détecter par palpation, dans la position détachée, l'emplacement et/ou la position de l'os.

2. Support d'usinage selon la revendication 1, dans lequel le dispositif d'ajustement comporte trois degrés de liberté d'ajustement.

3. Support d'usinage selon la revendication 1 ou 2, dans lequel le dispositif d'ajustement est réalisé pour la rotation autour de trois axes.

4. Support d'usinage selon l'une quelconque des revendications 1 à 3, dans lequel le dispositif d'ajustement est réalisé pour un mouvement de translation.

5. Support d'usinage selon l'une quelconque des revendications 1 à 4, dans lequel le dispositif d'ajustement (300) est conçu de telle sorte qu'un mouvement relatif entre le premier et le deuxième bâti suivant au moins un degré de liberté d'ajustement est bloqué lorsqu'une force est exercée sur le premier ou le deuxième bâti, mais est possible lorsqu'un élément d'ajustement du dispositif d'ajustement est utilisé.

6. Support d'usinage selon l'une quelconque des revendications 1 à 5, dans lequel le dispositif d'ajustement est conçu de manière à pouvoir permuter entre une position ajustable et une position non ajustable, en particulier au moyen de forces pneumatiques ou hydrauliques.

7. Support d'usinage selon l'une quelconque des revendications 1 à 6, dans lequel le dispositif d'ajustement comporte des articulations rotatives, qui permettent un mouvement suivant au moins un degré de liberté.

8. Support d'usinage selon l'une quelconque des revendications 1 à 7, dans lequel le deuxième bâti est conçu de telle sorte qu'un bloc d'usinage (210) destiné à guider un mouvement de coupe ou de perçage est fixé sur le deuxième bâti, est réalisé d'un seul tenant avec celui-ci ou peut être monté fixe en position et, en particulier, de manière amovible sur celui-ci.

9. Support d'usinage selon l'une quelconque des revendications précédentes, dans lequel l'outil est un bloc de coupe, un gabarit de perçage, un outil de coupe ou un foret.

10. Support d'usinage selon l'une quelconque des revendications précédentes, dans lequel le dispositif d'ajustement présente une structure flexible, en particulier élastique, en particulier monobloc, dont la flexibilité est conçue de telle sorte que la déformation de la structure par des éléments d'ajustement correspond à des degrés de liberté d'ajustement prédéfinis.
